# EUROPÄISCHE PATENTANMELDUNG

(11) **EP 4 712 709 A1**
(43) Veröffentlichungstag der Anmeldung: **18.03.2026**
(21) Anmeldenummer: 24200710.2
(22) Anmeldetag: 17.09.2024
(51) Int. Cl.: H05K 7/20, G01N 33/28

(54) **DIREKTGEKÜHLTER UMRICHTER**

(71) Anmelder: Siemens Aktiengesellschaft, 80333 München (DE)
(72) Erfinder: Bigl, Thomas, 91074 Herzogenaurach (DE); Danov, Vladimir, 91056 Erlangen (DE); Grundmann, Jörn, 91091 Großenseebach (DE); Madkour, Sherif, 12159 Berlin (DE); Müller, Volker, 90459 Nürnberg (DE); Neugebauer, Stephan, 91058 Erlangen (DE); Reissner, Florian, 90411 Nürnberg (DE); Schwarz, Florian, 91466 Gerhardshofen (DE)
(74) Vertreter: Siemens Patent Attorneys

(57) **Zusammenfassung**

Die Erfindung betrifft einen direktgekühlten Umrichter (1), umfassend: einen Kühlkreislauf (5) zur Kühlung von Komponenten (2A, 2B, 2C) des Umrichters (1); ein Kühlfluid (7), das die Komponenten (2A, 2B, 2C) des Umrichters (1) wenigstens teilweise durchströmt und/oder umströmt und direkten Kontakt mit diesen hat; wenigstens einen Sensor (8), der in dem Kühlkreislauf (5) angeordnet ist, wobei der Sensor (8) dazu ausgelegt ist, Eigenschaften des Kühlfluids (7) zu erfassen.

## Beschreibung

Die Erfindung betrifft einen direktgekühlten Umrichter.

Leistungselektronische Bauteile von Umrichtern werden oftmals dezentral auf eine PCB platziert und mit Hilfe von Ventilatoren gekühlt. Die Luftkühlung hat den Vorteil, dass der Kühlfluid relativ einfach alle zu kühlende Bauteile erreichen kann. Nachteilig an der Luftkühlung ist z. B., dass Lüfter benötigt werden, die Lärm verursachen. Je nach Einsatzgebiet werden auch Filter für die Luft benötigt, somit ist mit einem höheren Druckverlust zu rechnen. Die Filter müssen zudem ausgetauscht werden.

Neuere Entwicklungen gehen verstärkt von direktgekühlten Komponenten aus. Das bedeutet, die Komponenten werden direkt durch ein elektrisch nicht-leitendes Fluid gekühlt.

Um die Leistungselektronik langlebig nutzen zu können, ist es wichtig zu wissen, wie lang die Restlebensdauer der einzelnen Komponenten ist und wann Kühlmittel getauscht werden müssen.

Der Erfindung liegt die Aufgabe zugrunde, dies zu ermöglichen.

Die Lösung der Aufgabe gelingt durch Anspruch 1, d.h. einen direktgekühlten Umrichter, umfassend, einen Kühlkreislauf zur Kühlung von Komponenten des Umrichters, ein Kühlfluid das die Komponenten des Umrichters wenigstens teilweise durchströmt und/oder umströmt und direkten Kontakt mit diesen hat, wenigstens einen Sensor, der in dem Kühlkreislauf angeordnet ist, wobei der Sensor dazu ausgelegt ist, Eigenschaften des Kühlfluids zu erfassen.

Das Kühlfluid ist vorteilhaft ein elektrisch nichtleitendes Fluid. Besonders bevorzugt ist hierbei ein biologisch abbaubares Öl, welches im Kühlfluid enthalten ist. Das Kühlfluid kann auch gänzlich aus einem biologisch abbaubaren Öl bestehen. Beispielsweise kann Midel 7131 eingesetzt werden.

Dadurch, dass das Öl biologisch abbaubar ist, können Umweltschäden vermieden werden, falls es zu Leckagen kommt.

Ferner ist auch ein weiterer Vorteil durch die biologische Abbaubarkeit gegeben, da eine Reduzierung von Eindämmungsmaßnahmen vorgenommen werden kann. Da bei einer Leckage keine Umweltschäden zu befürchten sind, kann als Sicherheit des Gehäuses hinsichtlich Leckagen herabgesetzt werden.

Vorteilhaft ist eine Ausführungsform, wonach der Sensor in Nachbarschaft zu einer Kreislaufpumpe angeordnet ist. Auf diese Weise können die Eigenschaften des Kühlfluids besonders gut erfasst werden.

Vorteilhaft erfasst der Sensor die Eigenschaften des Kühlfluids spektrotopisch, chemisch und/oder physikalisch.

Mehrere Eigenschaften des Kühlfluids können erfasst werden. Vorzugsweise werden diese gleichzeitig erfasst. Erfasst werden, insbesondere Wassergehalt, Oxidation, Gesamtbasenzahl (TBN), Temperatur und/oder Rußgehalt.

Ein erhöhter Rußgehalt liefert, beispielsweise Informationen über partielle Kurzschlüsse oder partiell erhöhte Temperaturen.

Ein erhöhter Wassergehalt deutet beispielsweise auf Undichtigkeiten im Umrichtersystem hin. Eine erhöhte Oxidation deutet beispielsweise auf zu viele Sauerstoffmoleküle hin, was auf eine Alterung des Kühlfluids, insbesondere des Öls, schließen lässt. Eine zusätzliche Temperaturmessung kann Rückschlüsse über die Fahrweise des Umrichters liefern und über eine noch verbleibende Restlebensdauer geben.

Vorteilhaft ist zudem eine Ausführungsform, wonach ein System zur Datenerfassung und/oder Datenanalyse ausgebildet ist, dass vorzugsweise auf künstlicher Intelligenz basiert. Das System ist zum Sammeln von Daten aus einer Mehrzahl an Umrichtern und/oder zur Vorhersage der Lebensdauer eines oder auch mehrerer Umrichter und/oder zur Vorhersage eines Kühlfluidwechsels eines notwendigen Kühlfluidwechsels ausgebildet.

Ein notwendiger Austausch einer Komponente basierend auf den gesammelten Daten kann auch vorteilhaft vorhergesagt werden. Die künstliche Intelligenz ist vorteilhaft zur Validierung in Echtzeit durch begleitende Simulationen oder Auswertungen weiterer Performancecharakteristiken des Umrichters, beispielsweise des Wirkungsgrads, ausgebildet.

In Produktionsbetrieben ist die Ausfallsicherheit sehr wichtig. Systeme werden redundant installiert. Bei Ausfall eines Systems übernimmt das nächste. Nichtsdestotrotz müssen Systeme gewartet werden. Oftmals werden deshalb Zeiträume bestimmt, in denen Wartungen stattfinden. Wenn ein Monitoring-System vorhersagen kann, wann ein leistungselektronisches Bauteil kaputt geht, kann man die Wartung entsprechend geplant werden. Ein unterbrechungsfreier Betrieb ist somit möglich. Durch den Erhalt von realen Kundenbetriebsdaten, können die Bedingungen ermittelt werden, die einen Ausfall der Bauteile begünstigen oder verursachen.

Diese Daten können durch KI interpretiert werden und auf dieser Grundlage Vorhersagen für bestimmte Bauteile getroffen werden.

Weitere Vorteile der Erfindung sind: Lärm durch Lüfter wird durch die Erfindung vermieden. Zudem werden keine Filter für den Lüfter benötigt.

Die Lösung der Aufgabe gelingt ferner durch ein Verfahren zur Überwachung eines direktgekühlten Umrichters, wobei mittels eines Sensors Eigenschaften des Kühlfluids erfasst werden. Die Eigenschaften werden vorteilhaft spektroskopisch, chemisch und/oder physikalisch erfasst, vorzugsweise werden die Eigenschaften des Kühlfluids gleichzeitig erfasst. Erfasst werden insbesondere Wassergehalt, Oxidation, Gesamtbasenzahl (TBN), Temperatur und/oder Rußgehalt. Die erfassten Eigenschaften werden vorteilhaft mit definierten Werten der jeweiligen Eigenschaft verglichen. Ein Indikator wird vorteilhaft dann ausgegeben, wenn ein Wert der erfassten Eigenschaft höher liegt als der vorher definierte Wert.

Ein erhöhter Rußgehalt dient hierbei vorzugsweise als Indikator für partielle Kurzschlüsse oder partiell erhöhte Temperaturen. Ein erhöhter Wassergehalt dient hierbei als Indikator für Undichtigkeiten. Eine erhöhte Oxidation dient vorteilhaft als Indikator für die Alterung des Kühlfluids. Eine erhöhte Temperatur dient vorteilhaft als Indikator für eine Fahrweise des Umrichters und/oder eine verbleibende Restlebensdauer.

Vorteilhaft können auf Basis von künstlicher Intelligenz und aus den gesammelten Daten, Rückschlüsse über die Lebensdauer und Kühlfluidwechsel erlangt werden. Ein Austausch einzelner Komponenten kann basierend auf diesen gesammelten Daten empfohlen werden.

Der Einbau des Sensors kann beispielsweise in der Kühlmittelleitung vor der Pumpe eingebaut werden?. So kann die Überwachung der Kühlmitteleigenschaften besonders gut gewährleistet werden und mögliche Beschädigung des Umrichters können rechtzeitig ermittelt werden. Zusätzlich können die genannten KI-Ansätze genutzt werden, um aus den Daten zu lernen und geeignete Vorschläge für das Auswechseln von Komponenten zu unterbreiten.

Im Folgenden wird die Erfindung anhand der Figuren in den Ausführungsbeispielen beschrieben und erläutert.

Es zeigen:
- FIG 1: einen Umrichter mit Kühlkreislauf,
- FIG 2: ein Verfahren und
- FIG 3: ein System zur Datenerfassung und/oder Datenanalyse.

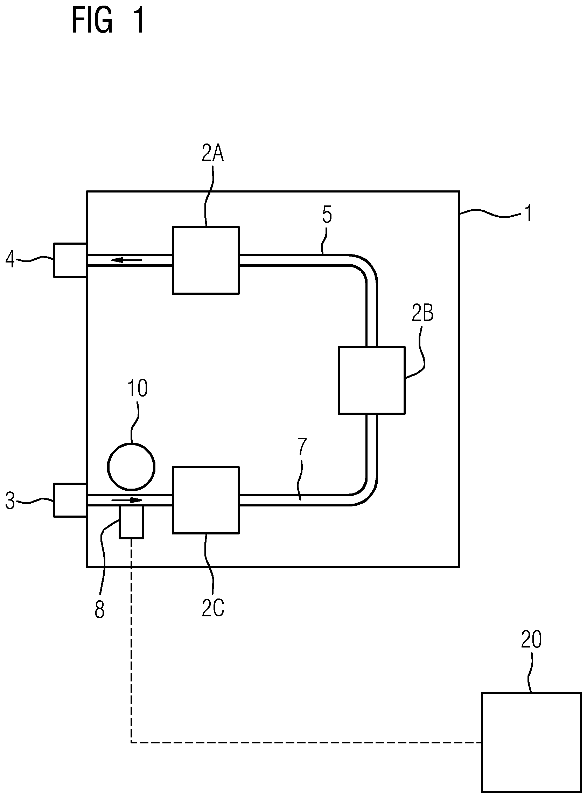

FIG 1 zeigt einen Umrichter 1 sowie einen Kühlkreislauf 5.

Ein Kühlfluid 7 kann beispielsweise an einer Einlassstelle 3 in den Umrichter 1 eingelassen werden und an einer Auslassstelle 4 wieder austreten. Das Kühlfluid 7 durchströmt und/oder umströmt in der Figur die Komponenten 2A, 2B und 2C und hat direkten Kontakt mit diesen. Der Kühlkreislauf 5 führt vorteilhaft durch Komponenten des Umrichters oder an diesen vorbei, dies dient der Kühlung der Komponenten.

FIG 1 zeigt ferner einen Sensor 8 der in dem Kühlkreislauf 5 angeordnet. Der Sensor 8 ist dazu ausgelegt, Eigenschaften des Kühlfluids 7 zu erfassen. Der Sensor 8 ist vorteilhaft in Nachbarschaft zu einer Kreislaufpumpe 10 angeordnet.

FIG 1 zeigt ferner ein System 20 zur Datenerfassung und/oder Datenanalyse.

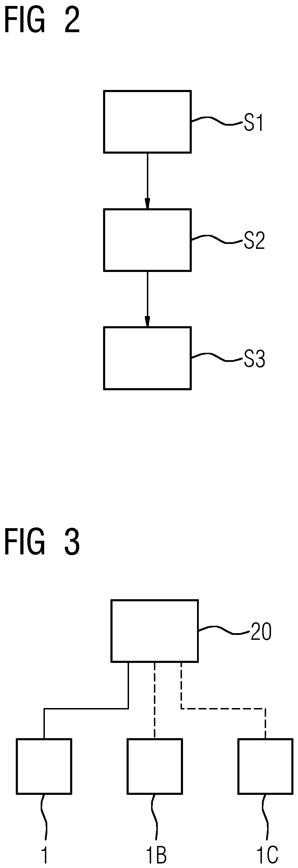

Durch das System 20 können Daten gesammelt werden, auch aus einer Mehrzahl an Umrichtern 1, 1B, 1C, siehe FIG 3.

So können auch auf Basis von KI notwendige Kühlfluidwechsel vorhergesagt werden. Auch eine Vorhersage der Lebensdauer, wenigstens eines Umrichters 1, 1B, 1C ist möglich.

Der Sensor 8 umfasst die Eigenschaften des Kühlfluids 7 vorteilhaft spektroskopisch, chemisch und/oder physikalisch. Der Sensor 8 erfasst vorteilhaft Wassergehalt, Oxidation, Gesamtbasenzahl (TBN), Temperatur und/oder Rußgehalt im Kühlfluid 7.

FIG 2 zeigt ein Verfahren.

In einem Verfahrensschritt S1 werden die Eigenschaften des Kühlfluids 7 erfasst.

In einem Verfahrensschritt S2 werden die erfassten Eigenschaften mit definierten Werten der jeweiligen Eigenschaft verglichen.

In einem Verfahrensschritt S3 wird ein Indikator ausgegeben, wenn ein Wert der erfassten Eigenschaft höher liegt als der definierte Wert.

Nicht dargestellt aber dennoch im weiteren Verfahren möglich, ist beispielsweise eine Empfehlung zum Austausch einer Komponente auf Basis der gesammelten Daten.

Vorteilhaft an der Erfindung, dass keine Lüfter benötigt werden und somit ein geräuscharmer Betrieb möglich ist.

Je nach Einsatzgebiet werden Luftfilter benötigt. Ein damit verbundener Druckverlust stellt kein Problem mehr dar. Durch das Vermeiden von Lüftern muss keine Wartung und kein Austausch dieser stattfinden.

Die vorgeschlagene Kühlung ist zudem bezüglich einer Leistungsdichte (kW/m³) der Gesamteinheit vorteilhaft.

## Patentansprüche

1. Direktgekühlter Umrichter (1), umfassend:
- einen Kühlkreislauf (5) zur Kühlung von Komponenten (2A, 2B, 2C) des Umrichters (1),
- ein Kühlfluid (7), das die Komponenten (2A, 2B, 2C) des Umrichters (1) wenigstens teilweise durchströmt und/oder umströmt und direkten Kontakt mit diesen hat,
- wenigstens einen Sensor (8), der in dem Kühlkreislauf (5) angeordnet ist, wobei der Sensor (8) dazu ausgelegt ist, Eigenschaften des Kühlfluids (7) zu erfassen.

2. Direktgekühlter Umrichter (1) nach Anspruch 1, wobei das Kühlfluid (7) ein biologisch abbaubares Öl aufweist.

3. Direktgekühlter Umrichter (1) nach ein der vorhergehenden Ansprüche, wobei der wenigstens eine Sensor (8) in Nachbarschaft zu einer Kreislaufpumpe (10) angeordnet ist.

4. Direktgekühlter Umrichter (1) nach ein der vorhergehenden Ansprüche, wobei der Sensor (8) die Eigenschaften des Kühlfluids (7) spektroskopisch, chemisch und/oder physikalisch erfasst.

5. Direktgekühlter Umrichter (1) nach ein der vorhergehenden Ansprüche, wobei der Sensor (8) dazu ausgelegt ist, mehrere Eigenschaften des Kühlfluids (7), vorzugsweise gleichzeitig, zu erfassen, insbesondere Wassergehalt, Oxidation, Gesamtbasenzahl (TBN), Temperatur und/oder Rußgehalt.

6. Direktgekühlter Umrichter (1) nach ein der vorhergehenden Ansprüche, ferner aufweisend wenigstens ein System (20) zur Datenerfassung und/oder Datenanalyse, das vorzugsweise auf künstlicher Intelligenz (KI) basiert, ausgebildet zum Sammeln von Daten aus einer Mehrzahl an Umrichtern (1, 1B, 1C) und/oder zur Vorhersage der Lebensdauer eines Umrichters (1) und/oder zur Vorhersage eines notwendigen Kühlfluidwechsels.

7. Direktgekühlter Umrichter nach Anspruch 6, derart ausgebildet, dass ein notwendiger Austausch einer Komponente (2A, 2B, 2C) basierend auf den gesammelten Daten vorhersagbar ist.

8. Direktgekühlter Umrichter nach Anspruch 6 oder 7, wobei die KI zur Validierung in Echtzeit durch begleitende Simulationen und/oder Auswertungen weiterer Performance-Charakteristiken des Umrichters (1), beispielsweise des Wirkungsgrads, ausgebildet ist.

9. Verfahren zur Überwachung eines direktgekühlten Umrichters (1) nach einem der vorhergehenden Ansprüche, wobei mittels eines Sensors (8) Eigenschaften des Kühlfluids (7) erfasst werden.

10. Verfahren nach Anspruch 9, wobei die die Eigenschaften des Kühlfluids (7) spektroskopisch, chemisch und/oder physikalisch erfasst werden.

11. Verfahren nach einem der Ansprüche 9 oder 10, wobei, mehrere Eigenschaften des Kühlfluids (7), vorzugsweise gleichzeitig, erfasst werden, insbesondere Wassergehalt, Oxidation, Gesamtbasenzahl (TBN), Temperatur und/oder Rußgehalt.

12. Verfahren nach einem der Ansprüche 9 bis 11, wobei die erfassten Eigenschaften mit definierten Werten der jeweiligen Eigenschaft verglichen werden, wobei, wenn ein Wert der erfassten Eigenschaft höher liegt als der definierte Wert, ein Indikator ausgegeben wird.

13. Verfahren nach Anspruch 12, wobei ein erhöhter Rußgehalt als Indikator für partielle Kurzschlüsse oder partiell erhöhte Temperaturen dient, wobei ein erhöhter Wassergehalt als Indikator für Undichtigkeiten dient, wobei eine erhöhte Oxidation als Indikator für die Alterung des Kühlfluids (7) dient und/oder wobei eine erhöhte Temperatur als Indikator für eine Fahrweise des Umrichters (1) und/oder eine verbleibende Restlebensdauer dient.

14. Verfahren nach einem der Ansprüche 9 bis 13, wobei auf Basis von künstlicher Intelligenz aus den gesammelten Daten Rückschlüsse über Lebensdauer und Kühlfluidwechsel erlangt werden.

15. Verfahren nach Anspruch 14, wobei der Austausch einzelner Komponenten (2A, 2B, 2C) basierend auf den gesammelten Daten empfohlen wird.
